# EUROPEAN PATENT SPECIFICATION

(11) **EP 2 654 817 B1**
(45) Date of publication and mention of the grant of the patent: **25.02.2015**
(21) Application number: 11813361.0
(22) Date of filing: 21.12.2011
(51) Int. Cl.: A61L 27/48, A61L 27/52, A61L 27/18, A61K 9/00

(54) **COMPOSITE MATERIAL SUITABLE FOR BIOMEDICAL USE, AND METHOD TO PRODUCE IT**
VERBUNDSTOFF FÜR BIOMEDIZINISCHE VERWENDUNG UND VERFAHREN ZU SEINER HERSTELLUNG
MATÉRIAU COMPOSITE APPROPRIÉ POUR USAGE BIOMÉDICAL ET SON PROCÉDÉ DE FABRICATION

(30) Priority: 22.12.2010 IT UD20100233
(43) Date of publication of application: 30.10.2013
(73) Proprietor: Limacorporate SPA, 33038 San Daniele Del Friuli (IT)
(72) Inventor: SEGATTI, Francesco, I-33100 UDINE (IT); MERCURI, David, I-33100 UDINE (IT)
(74) Representative: Petraz, Gilberto Luigi
(86) International application number: PCT/IB2011/003102
(87) International publication number: WO 2012/085643

(56) References cited:
- EP-A1- 1 614 696
- EP-A2- 0 466 300

## Description

### FIELD OF THE INVENTION

The present invention concerns a composite material for biomedical uses, and the method to produce it, which provides to compatibilize a polymer fraction inside a three-dimensional reticulated matrix, such as gel.

For example, but not only, the material according to the present invention can be used as a biomedical device for treating osteoarthritis, in particular for the visco-supplementation of degenerated synovial liquid.

### BACKGROUND OF THE INVENTION

In the biomedical field, there is a need to develop new materials, in particular suitable for intra-articular application, which show a good compromise between elastic and viscous module. It is known that a biomedical material that has too high a value of elastic module is not suitable for application in the treatment of osteoarthritis, in particular for the visco-supplementation of degenerated synovial liquid, since it tends to fragment.

Moreover, if the biomedical material has too high a viscous module, it tends only to thicken the synovial liquid.

A versatile and frequently used biomaterial is hyaluronic acid, which is used in weakly reticulated preparations but which is expensive, rapidly degradable and with poor visco-elastic properties. Document EP-A-0.466.300 (EP'300) describes a biocompatible visco-elastic slurry gel, formed by a polymer gel with a hyaluronic acid base and a fluid phase formed by a water solution of a polymer, such as for example hyaluronic acid itself.

Polymers N-methyl-amides derivatives of carboxymethylcellulose, alginic acid or carboxymethyl starch, used in the biomedical field, are also known, from the European patent EP-B-1.614.696 (EP'696), in the name of the present Applicant.

These polymers, which have a semi-synthetic derivation, not animal or biotechnological, do not have any protein contaminants. However, neither EP'696 nor EP'300 supply any teaching on the rheological behavior of the polymers described therein, either reticulated or in solution.

Purpose of the present invention is to produce a material, and to perfect a relative method to produce it, which has a good compromise between elastic module and viscous module, so that it can be used in biomedical applications, in particular intra-articular.

The Applicant has devised, tested and embodied the present invention to overcome the shortcomings of the state of the art and to obtain these and other purposes and advantages.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

The word "comprise" and variants of the word such as "comprises" and "comprising" are used here to indicate the inclusion of a clearly expressed whole or clearly expressed wholes but not the exclusion of any other whole or any other wholes, unless in the context or in use an exclusive interpretation of the word is required.

### SUMMARY OF THE INVENTION

The present invention is set forth and characterized in the independent claims, while the dependent claims describe other characteristics of the invention or variants to the main inventive idea.

The present invention is based on what is described in EP'696, which is entirely incorporated here for reference.

In particular, the present invention uses the polymers N-methyl-amides derivatives of carboxymethylcellulose, alginic acid or carboxymethyl starch as in EP'696 as a starting product to achieve a composite material suitable for biomedical use, in particular for use in the treatment of osteoarthritis, even more particularly for the visco-supplementation of degenerated synovial liquid.

Following experiments carried out by Applicant, the products have shown unexpected chemical-physical, rheological and biological properties that satisfy the following requirements:
- their behavior is reproducible and modulatable in terms of viscosity, swelling and visco-elasticity of water solutions/dispersions, comparable with that of hyaluronic acid or its functionalized and/or reticulated derivatives;
- they can be worked in various forms, such as filaments, tissues, spreadable or injectable gel;
- easily sterilized in the form of raw products, semi-worked or finished;
- low cost of the original materials and derivatives, including the reticulates, therefore making the products suitable for use in the field of foodstuffs and pharmaceuticals, the medical and cosmetic fields. The composite material according to the present invention comprises a first phase consisting of a three-dimensional matrix of a reticulated hydrogel deriving from one of the polymers N-methyl-amides derivatives of carboxymethylcellulose, alginic acid or carboxymethyl starch, and a second phase that comprises one of said polymers.

In particular, the material according to the present invention is obtained starting from a dispersion of highly reticulated hydrogel deriving from one of the above polymers in one of the above polymers, for example in powder form of one of the above polymers, or in a solution, in which the solvent is mainly water, of one of the above polymers.

In a first form of embodiment, the reticulated hydrogel is in dried powder form.

In a second form of embodiment, the reticulated hydrogel is in solution.

In some forms of embodiment, the degree of occupation of the carboxyl functions of the hydrogel is advantageously greater than about 70%, that is, up to 50% amidation of the polymer chains and at least 20% of reticulation of the functional groups of the polymer that are still free.

In one form of embodiment, in the composite material according to the present invention there are from 2 to 5, preferably from 2.5 to 4.5, even more preferably from 3 to 4 parts of reticulated hydrogel (first phase) and from 5 to 8, preferably from 5.5 to 7.5, even more preferably from 6 to 7 parts of polymer as such (second phase), with the provision that the sum of the parts of the two phases, on a weight basis, is equal to 10.

Some forms of embodiment provide that the ratio between the first phase and the second phase, on a weight basis, in the composite material according to the present invention, can be: 2:8, 2.5:7.5, 3:7, 3.5:6.5, 4:6, 4.5:5.5, 5:5.

In particular, in the above polymers the percentage of N-methyl-amide of the original carboxyl groups in the polysaccharide (AD, substitution or amidation degree) is less than 100%.

Preferably, the same polymer is used for both the first phase and the second phase which form the composite material, selecting on each occasion, among the N-methyl-amide of carboxymethylcellulose, alginic acid or carboxymethyl starch, the polymer suitable to make the desired biomedical device.

The method according to the present invention provides that one of the polymers is prepared and purified, obtaining a polymer solution, for example according to one of the methods described in EP'696, or by means of other purification techniques, such as tangential filtration. Purification is advantageously greater than 99%.

Advantageously, the preparation and purification performed provides to obtain a polymer solution that contains from 1% to 2% in weight, preferably from 1.25% to 1.75% in weight, of a selected one of the polymers.

This concentration of the polymer solution is advantageous because it represents a compatible material useful for obtaining a viscous module greater than 75Pa (2.5 Hz) compared with a lower enzymatic degradability, for example Hyaluronidase (Type 1), if compared with an analogous solution of hyaluronic acid.

Subsequently, according to a first form of embodiment of the method according to the present invention, the polymer solution is concentrated by means of partial dehydration.

In this first form of embodiment, a first fraction or part of the concentrated polymer solution is subjected to an operation in which the solvent is totally removed by means of a drying technique, for example freeze-drying, and then subjected to grinding and sieving in order to obtain particles of dried powder that have a homogeneous distribution of the nominal diameter, preferably a nominal diameter less than or equal to about 2 mm, for example 1 mm, 1.5 mm or 2 mm, to prepare the first phase.

A second fraction or part of the concentrated polymer solution is subjected to reticulation, for example according to one of the methods described in EP'696, obtaining the reticulated hydrogel, which is subsequently purified by washing, in this case too, for example, as described in EP'696, or using other alternative techniques.

In a first variant, the reticulated hydrogel is then concentrated and the washing liquid used in the purification is totally removed, for example by freeze-drying.

Then the reticulated and concentrated hydrogel is subjected to grinding and and sieving in order to obtain particles of dried powder that have a homogeneous distribution of the nominal diameter in order to obtain said second phase, preferably a nominal diameter less than or equal to about 2 mm, for example 1 mm, 1.5 mm or 2 mm.

In particular, in this variant form of embodiment, for the purposes of biomedical use, a desired quantity of the powdered polymer material thus obtained, and a desired quantity of the powdered gel material are hydrated in a physiological solution, or sterile water.

In a second variant of the first form of embodiment of the present invention it is possible to disperse a desired quantity of powdered gel in the polymer solution previously prepared. In this second variant form of embodiment, the particles of hydrogel are dispersed in the polymer solution. In particular, in this form of embodiment, the dehydrated hydrogel is put into contact with the polymer solution, thus enabling the polymer to penetrate inside the three-dimensional reticular structure of the hydrogel. The process can also occur dynamically, for example using a closed rotating system (dynamic compatibilization). The re-hydration of the hydrogel by means of the polymer solution allows the chains to migrate inside the lattice.

The first and second variant of the first form of embodiment have the advantage that they allow an exact control of the relative quantities of the two components and hence to obtain a highly homogeneous final product.

In a second form of embodiment of the present invention the purified gel, still in solution, is compatibilized with the polymer solution so that the polymer penetrates inside the three-dimensional reticular structure of the hydrogel. The process occurs dynamically, for example using a closed rotating system (dynamic compatibilization). The re-hydration of the hydrogel and the polymer solution allows the chains to migrate inside the lattice.

Subsequently, in the second form of embodiment, the solvent is removed, for example by freeze-drying or drying, and the overall product obtained is ground and sieved so as to obtain the powdered particles with desired nominal sizes, preferably less than or equal to about 2 mm, for example 1 mm, 1.5 mm or 2 mm.

In particular, in the second form of embodiment, for biomedical use, a desired quantity of the powdered material thus obtained is dispersed in a physiological solution, or in sterile water, so that it can subsequently be injected.

In any case, for both forms of embodiment as described above, the nominal size of the particles obtained according to the present invention is selected as particularly suitable and specific for intra-articular injection.

The composite material according to the present invention, once suitably hydrated and packaged, can advantageously be subjected to sterilization, for example using steam.

The present invention provides an optimal compromise between the values of elastic module, to which the reticulated component of the material contributes, and which has the effect of dissipating the loads exerted during use on the joints, and of the viscous module, to which the polymer component of the material contributes.

The composite material obtained with the present invention has the property that it is visco-elastic in particular due to the effect of two factors:
- the presence of fragments of hydrogel inside the solution to be injected confers consistency, mechanical resistance and ability to absorb shocks;
- the polymer with high molecular weight incorporated inside the hydrogel increases the consistency of the hydrogel and increases the viscosity of the water.

Whether the two phases are ground and sieved independently of each other and compatibilized only at the end, or are compatibilized at the beginning by diffusion in a water environment and then subsequently ground and sieved together, the composite material obtained can be packaged and conserved in a package, such as an envelope, bag, syringe, phial or similar container for biomedical use, of the sterile type.

All in all, the final composite material contains, in a water or physiological solution suitable for supplementation for example by means of injection, from 1.75% to 5% in weight, preferably from 2% to 3.75% in weight, for example about 3.61%, of a selected one of the polymers and the reticulated hydrogel deriving from one of the polymers.

### EXAMPLE 1

A polymer N-methyl-amides derivatives of carboxymethylcellulose, alginic acid or carboxymethyl starch is produced as described in EP'696. The polymer produced is in a water solution, for example about 1.5% in weight.

Depending on whether the first or second form of embodiment of the present invention is used, the polymer solution can be concentrated, for example by means of a rotary drier.

According to the first variant of the first form of embodiment of the present invention, the solvent is totally removed from a first part of the concentrated polymer solution by means of drying, for example freeze-drying.

Furthermore, a second part of the concentrated polymer solution produced is subjected to reticulation, as described in EP'696, to obtain a hydrogel that is purified by means of successive washings in a saline solution 0.1M or in water. Alternatively, other purification techniques may be used.

Then the swelling water is totally removed from the hydrogel by means of a suitable process, such as drying and/or freeze-drying.

The two dried phases of polymer and reticulated hydrogel are subjected to grinding and sieving, separately from each other, so as to obtain powders of the two phases with a nominal diameter equal to about 2 mm. The two powders of the first and second phase can be dispersed separately from each other in a water or physiological solution and then compatibilized when they are introduced into the injection member.

On the contrary, in the second variant of the first form of embodiment of the present invention, the hydrogel in the form of solid powder obtained is put into contact with the polymer solution as previously prepared, obtaining the incorporation of the polymer in the lattice. The water present in the sample is then eliminated and when this operation is complete the product is ground so as to obtain a compound with homogeneous sizes and equal to about 2 mm nominal diameter.

Or, according to the second form of embodiment of the present invention, it is also possible to compatibilize the purified gel still in solution in the polymer solution as previously prepared.

The ratio between the reticulated hydrogel and the powder used is respectively 2 to 3. Another example of the ratio between the reticulated hydrogel and the polymer is 3 to 7.

### EXAMPLE 2

The powdered composite material in example 1 is dispersed in sterile water, for example about 150 mg of composite material in about 4 ml of solution. Once hydrated, the hydrogel swells, allowing the polymer to spread into the surrounding environment for the desired effect of intra-articular treatment.

### COMPARATIVE EXAMPLE 1

Applicant has carried out a rheological characterization of the material of the present invention, comparing it with a material formed completely by one of the polymers N-methyl-amides derivatives of carboxymethylcellulose, alginic acid or carboxymethyl starch (comparative sample 10, 100% polymer), and also with a material formed completely by a reticulated hydrogel deriving from said polymers (comparative sample 0, 100% reticulate).

Eleven samples were prepared in water solution, with a concentration of 3.61% w/w, containing different polymer/gel ratios. Sample zero (0) has a single gel component, sample one (1) has a polymer/gel ratio of 1/9 and so on, until sample ten (10) that has a polymer component only.

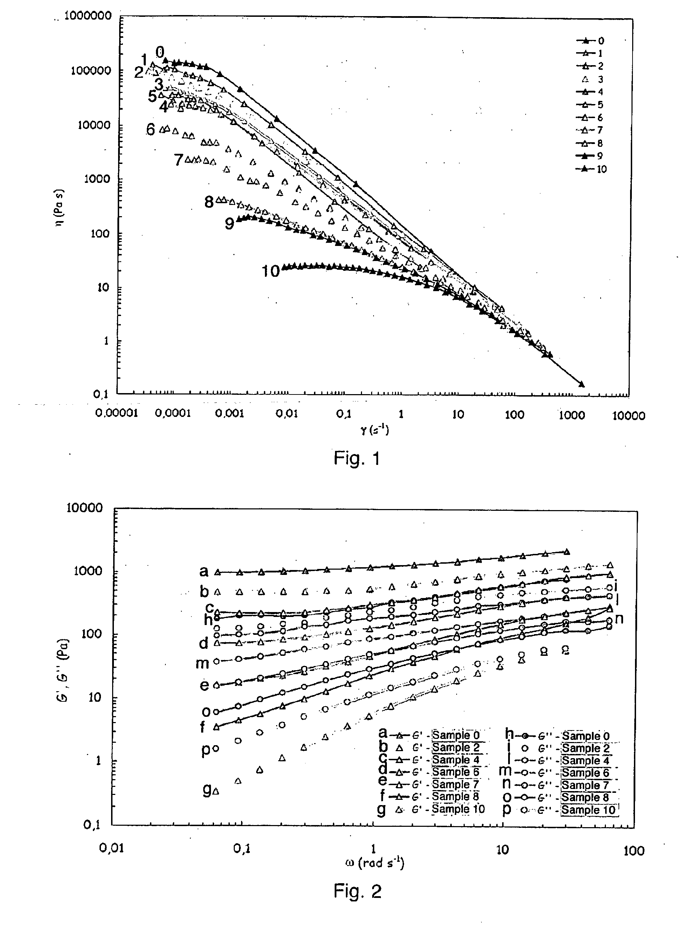

The results of the comparison are shown in fig. 1, which shows a graph of the stationary viscosity (η [Pa*s] on the y-axis) as a function of the speed of deformation (γ [s⁻¹] on the x-axis), and in fig. 2 which shows a graph of the mechanical spectra (ω [rad*s⁻¹] on the x-axis, G', G", [Pa] on the y-axis) for the eleven samples analyzed.

The comparative sample 10 showed a behavior substantially comparable to a non-Newtonian fluid, that is, the pseudo-plastic type.

The comparative sample 0 showed a high elastic module and low viscous module, with the disadvantages that that entails.

The sample according to the present invention, on the contrary, has a behavior similar to pseudo-plastic but with a good compromise between elastic and viscous module.

### COMPARATIVE EXAMPLE 2

Applicant has carried out a test, comparing the degradation of the hyaluronic acid polymer (Hyal) with a polymer according to the present invention, in this case a polymer N-methyl-amides derivatives of carboxymethylcellulose (CMC-A), by means of an enzyme present inside the human organism on an articular level, especially in the event of inflammation, for example Hyaluronidase (Type 1). The comparison was carried out on the polymeric component since, according to literature, it is known that the reticulated component reduces degradation times.

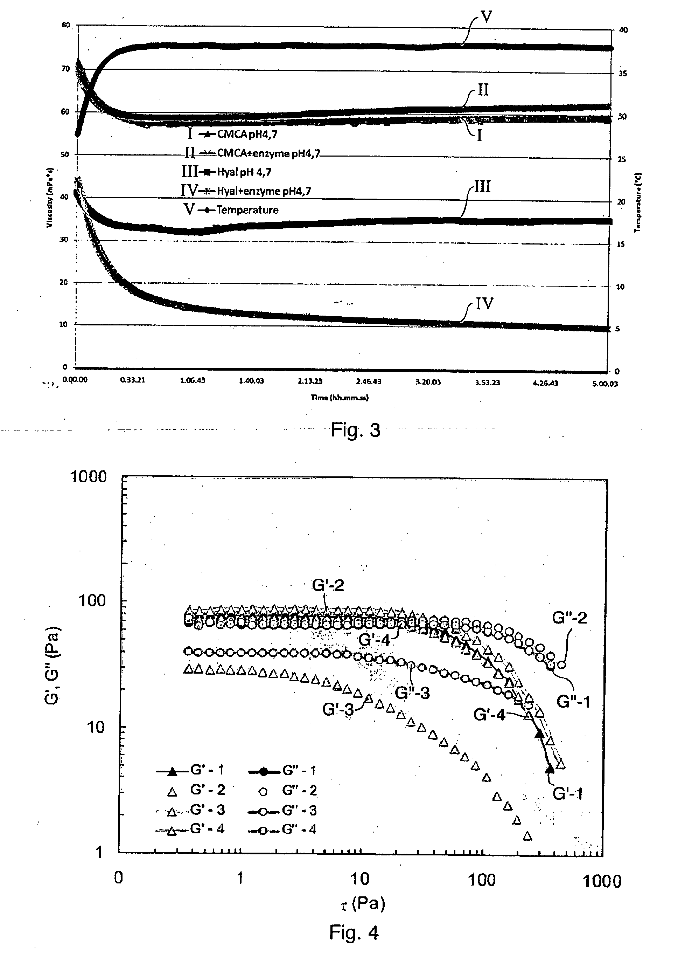

The test was carried out evaluating the viscosity of the solution over time, at a temperature of 37°C and with a pH of 4.7, since the viscosity of the polymer depends on the molecular weight, and the enzyme acts by reducing the length of the polymeric chain and consequently the weight. The test was carried out for both types of polymer both with and without enzyme, in the same conditions, and the results obtained are shown in fig. 3. While the hyaluronic acid, in the presence of the enzyme, shows a consistent loss of viscosity already after 30 minutes, equal to 50%, the polymer according to the present invention shows the same development both with and without the enzyme, in the same conditions, and therefore the enzyme has no degradation effects on the polymer in the time considered (and up to 24 hours).

### COMPARATIVE EXAMPLE 3

Applicant has carried out a test to compare the mechanical properties by means of a rheometric analysis of samples 1, 2, 3 and 4 of composite material according to the present invention, prepared as described in Example 1.

In particular, a first group of components 1, 2 and 4 was prepared according to the first form of embodiment of the present invention, using different techniques for grinding the reticulated gel.

A second component 3 was instead prepared according to the second form of embodiment of the present invention, that is, omitting the step of concentrating the hydrogel, for example by means of a rotary evaporator, carried out before freeze-drying.

This comparison emphasizes the advantages, for the purposes of the mechanical properties of the final product, of the operation of preparing the ground and sieved powder of gel which is compatibilized with polymer powder or polymer solution.

The differences between the samples emerge to a similar extent from the oscillatory tests, both from scanning the stresses and from scanning the frequencies, as can be seen in fig. 4. The deviation from the linear viscoelastic behavior occurs with lower stresses in the case of sample 3. The mechanical profiles of the samples are similar to each other: the traces of the two modules are very close, and almost rectilinear in the region of the intermediate frequencies, whereas they diverge at lower frequencies, which indicates a visco-elastic behavior just beyond the sol-gel transition. Compared with the other samples, sample 3 is in a band of lower values of the two modules, since the difference is greater at low frequencies.

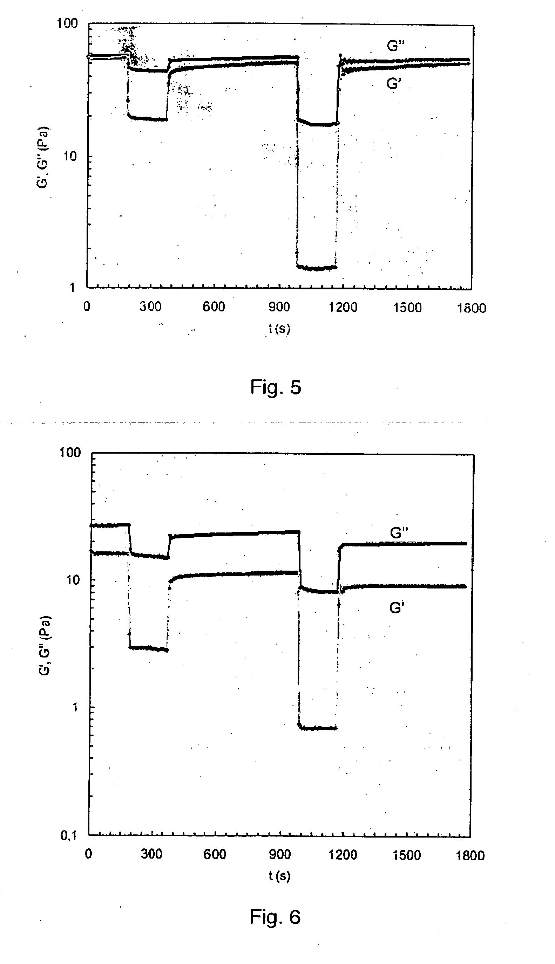

Figs. 5 and 6 show the results of the recovery test carried out completely in conditions of oscillatory motion at fixed frequency (1 Hz), interposing segments with a greater oscillation amplitude (100 Pa and 500 Pa) than the reference segments at low stress (1 Pa). The results obtained for sample 1 are similar to those of samples 2 and 4, and differ from those of sample 3. In the first case, it can be observed that even when the oscillations with a very wide amplitude produce a substantial reduction of the modules, in particular of G', there is a substantial recovery of both modules in the following segment. On the contrary, in the case of sample 3, the de-structuration is obvious and the recovery is less, in particular in the last segment.

A recovery test after a segment with a constant deformation speed (100 s⁻¹) was made on the standard system: 66% for G' and 80% for G", which percentage is drastically reduced when no concentration by drying is carried out on the gel.

### COMPARATIVE EXAMPLE 4

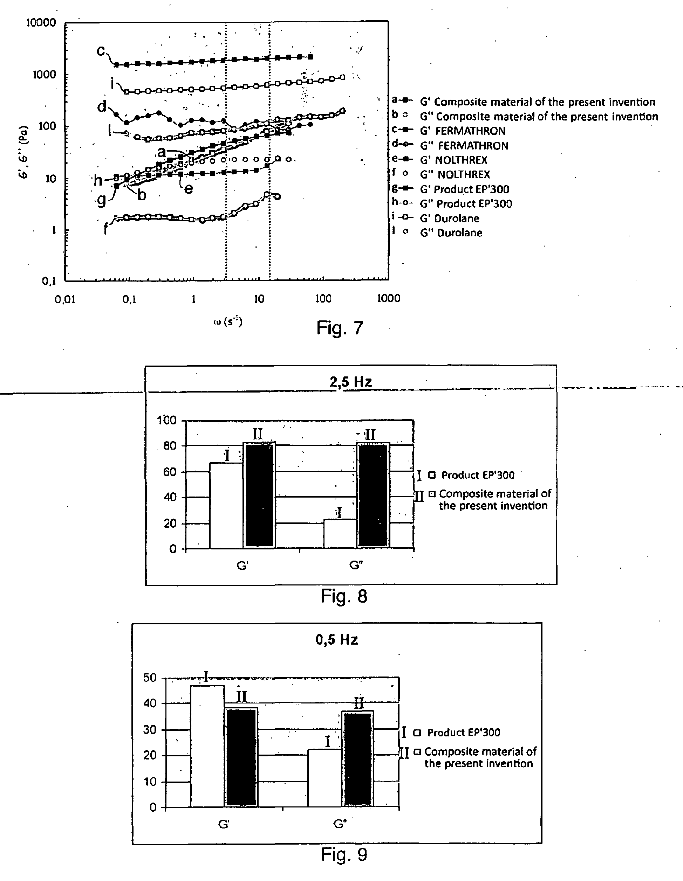

Applicant carried out a test to compare the mechanical characteristics by means of a rheometric analysis of the composite material prepared according to the first form of embodiment of the present invention, and some known products present on the market, including Fermathron, Nolthrex, Durolane and products deriving from EP'300 (fig. 7).

Among the known products present on the market, products deriving from EP'300 and that developed by Applicant (whose G' and G" values are indicated by A and b in fig. 7) are those in a central response band, supplying behaviors comparable with those of the synovial fluid.

Applicant also compared the rheological behavior of the product deriving from EP'300 with the one developed by Applicant (figs. 8 and 9).

From this comparison it emerges that there is a better performance in terms of visco-elasticity at high frequencies (quick walk/run) and a behavior substantially comparable to low frequencies (slow walk).

From the comparative examples reported above, it can be concluded that the material according to the present invention generally has the following advantageous properties:
i) elastic module G' greater than 31 Pa and viscous module G" greater than 32 Pa, recorded at a frequency of 0.5 Hz;
ii) elastic module G' greater than 70 Pa and viscous module G" greater than 70 Pa, recorded at a frequency of 2.5 Hz;
iii) recovery capacity after a segment with a constant speed of deformation (100 s-1): equal to 66% for G' and 80% for G".

All the results were recorded after injection of the product by means of a syringe with an 18G needle or in conditions similar to conditions of use.

## Claims

1. Composite material suitable for biomedical use, **characterized in that** it comprises a first phase consisting of a reticulated hydrogel deriving from a polymer chosen from among polymers N-methyl-amides derivatives of carboxymethylcellulose, alginic acid or carboxymethyl starch and a second phase which comprises one of said polymers.

2. Material as in claim 1, **characterized in that** it comprises from 2 to 5, preferably from 2.5 to 4.5, even more preferably from 3 to 4 parts of reticulated hydrogel and from 5 to 8, preferably from 5.5 to 7.5, still more preferably from 6 to 7 parts of polymer as such, with the provision that the sum of the parts of the two phases, on a weight basis, is equal to 10.

3. Material as in claim 2, **characterized in that** the ratio between the first phase and the second phase, on a weight basis, is selected between: 2:8, 2.5:7.5, 3:7, 3.5:6.5, 4:6, 4.5:5.5, 5:5.

4. Material as in claim 1, 2 or 3, **characterized in that** the degree of occupation of the carboxyl functions of the hydrogel is greater than about 70%.

5. Material as in any claim hereinbefore, **characterized in that** the polymer of the second phase is the same reticulated polymer as the first phase.

6. Material as in any claim hereinbefore, **characterized in that** the solution of the second phase contains from 1 % to 2% in weight, preferably from 1.25% to 1.75% in weight, of a polymer selected from among said polymers.

7. Material as in any claim hereinbefore, **characterized in that** it contains in all from 1.75% to 5% in weight, preferably from 2% to 3.75% in weight, of one of said polymers and of said reticulated hydrogel deriving from one of said polymers.

8. Material as in any claim hereinbefore, **characterized in that** the first phase and the second phase are formed by particles of dried powder with a homogeneous distribution of the nominal diameter less than or equal to about 2 mm.

9. Material as in any claim hereinbefore, **characterized in that** it comprises a water or physiological solution in which the first and second phase are dispersed.

10. Product comprising a sterile confection containing a composite material as in any claim hereinbefore.

11. Medical device comprising a dispersion of a desired quantity of the composite material as in any claim hereinbefore in a physiological or water solution.

12. Method to achieve a composite material suitable for biomedical use, **characterized in that** it provides to prepare a first phase comprising a reticulated hydrogel deriving from a polymer chosen from among polymers N-methyl-amides derivatives of carboxymethylcellulose, alginic acid or carboxymethyl starch, and a second phase which comprises one of said polymers.

13. Method as in claim 12, **characterized in that** it provides to prepare and purify one of said polymers in the form of a polymer solution, to prepare a dried powder of reticulated hydrogel from a part of said polymer solution and to associate said dried powder of reticulated hydrogel with a dried powder obtained from another part of said polymer solution, or directly with said other part of said polymer solution.

14. Method as in claim 13, **characterized in that** it provides:
- to concentrate the polymer solution by partial dehydration,
- to divide the concentrated polymer solution by partial dehydration into two fractions or parts,
- to totally remove the solvent from a first fraction or part,
- to subject the first fraction or part, from which the solvent has been removed, to grinding and sieving so as to obtain particles of dried powder that have a homogeneous distribution of the nominal diameter so as to obtain said first phase,
- to reticulate a second fraction or part of the concentrated polymer solution, obtaining the reticulated hydrogel which is subsequently purified by washing,
- to concentrate the reticulated hydrogel and remove the washing liquid used for purifying the hydrogel,
- to subject the concentrated reticulated hydrogel from which the washing liquid has been removed to grinding and sieving so as to obtain particles of dried powder that have a homogeneous distribution of the nominal diameter so as to obtain said second phase,
- to disperse in a water or physiological solution separately one from the other the first phase and the second phase and subsequently to put together the two solutions containing the first phase and the second phase.

15. Method as in claim 14, **characterized in that** it provides to prepare and purify one of said polymers in the form of a polymer solution, to prepare a reticulated hydrogel in solution from one part of said polymer solution and to associate said reticulated hydrogel in solution with another part of said polymer solution.

16. Method as in claim 15, **characterized in that** it provides:
- to reticulate a first part of the polymer solution, obtaining the reticulated hydrogel, which is subsequently purified by washing,
- to remove the washing liquid used in purifying the hydrogel, by drying it,
- to put a second part of the polymer solution in contact with the dried hydrogel, ensuring that the polymer is incorporated inside the reticular structure of the hydrogel,
- to remove the solvent,
- to grind the product obtained in order to obtain particles in powder with a homogeneous nominal distribution.

17. Method as in any claim from 13 to 16, **characterized in that** the polymer solution of one of said polymers prepared and used contains from 1% to 2% in weight, preferably from 1.25% to 1.75% in weight, of a selected one of said polymers.

18. Method to achieve a medical device for biomedical use which provides to disperse a desired quantity of the composite material as in any claim from 1 to 9, or obtainable as in any claim from 12 to 17, in a physiological solution or in sterile water.

## Patentansprüche

1. Verbundstoff zur biomedizinischen Verwendung, **dadurch gekennzeichnet, dass** er aufweist eine erste Phase aus einem vernetztem Hydrogel, welches aus einem Polymer, welches ausgewählt ist aus den Polymeren N-Methyl-Amid-Derivaten von Carboxylmethylcellulose, Alginsäure oder Carboxylmethylstärke, abgeleitet ist, und eine zweite Phase, welche einen dieser Polymere aufweist.

2. Stoff nach Anspruch 1, **dadurch gekennzeichnet, dass** er zu 2 bis 5, bevorzugt 2,5 bis 4,5, besonders bevorzugt 3 bis 4, Teilen vernetztes Hydrogel und zu 5 bis 8, bevorzugt 5,5 bis 7,5, besonders bevorzugt 6 bis 7, Teilen Polymer als solches umfasst, mit der Maßgabe, dass die Summe der Teile der beiden Phasen, auf Gewichtsbasis, gleich 10 ist.

3. Stoff nach Anspruch 2, **dadurch gekennzeichnet, dass** das Verhältnis zwischen der ersten Phase und der zweiten Phase, auf Gewichtsbasis, ausgewählt ist zwischen: 2:8, 2,5:7,5, 3:7, 3,5:6,5, 4:6 4,5:5,5, 5:5.

4. Stoff nach Anspruch 1, 2 oder 3, **dadurch gekennzeichnet, dass** der Sättigungsgrad der Carboxylgruppen des Hydrogels größer als 70% ist.

5. Stoff nach einem der vorstehenden Ansprüche, **dadurch gekennzeichnet, dass** der Polymer der zweiten Phase der gleiche vernetzte Polymer ist, wie jener der ersten Phase.

6. Stoff nach einem der vorstehenden Ansprüche, **dadurch gekennzeichnet, dass** die Lösung der zweiten Phase 1 Gew.-% bis 2 Gew.-%, bevorzugt 1,25 Gew.-% bis 1,75 Gew.-%, des aus den Polymeren ausgewählten Polymers umfasst.

7. Stoff nach einem der vorstehenden Ansprüche, **dadurch gekennzeichnet, dass** er insgesamt 1,75 Gew.-% bis 5 Gew.-%, bevorzugt 2 Gew.-% bis 3,75 Gew.-%, von einem der Polymere und von dem vernetzten Hydrogel, welches aus einem der Polymere abgeleitet ist, aufweist.

8. Stoff nach einem der vorstehenden Ansprüche, **dadurch gekennzeichnet, dass** die erste Phase und die zweite Phase durch Partikel getrockneten Pulvers mit einer homogenen Verteilung des Nenndurchmessers von kleiner oder gleich etwa 2 mm gebildet sind.

9. Stoff nach einem der vorstehenden Ansprüche, **dadurch gekennzeichnet, dass** es eine Wasser- oder physiologische Lösung, in welcher die erste und zweite Phase dispergiert sind, aufweist.

10. Produkt, welches eine sterile Konfektion, umfassend einen Verbundstoff nach einem der vorstehenden Ansprüche, aufweist.

11. Medizinische Vorrichtung, welche eine Dispersion einer gewünschten Menge des Verbundstoffs nach einem der vorstehenden Ansprüche in einer physiologischen oder Wasser-Lösung aufweist.

12. Verfahren zur Herstellung eines Verbundstoffs zur biomedizinischen Verwendung,
**dadurch gekennzeichnet, dass** es vorsieht, eine erste Phase, welche ein vernetztes Hydrogel aufweist, welches von einem Polymer, welches ausgewählt ist aus den Polymeren N-Methyl-Amid-Derivaten von Carboxylmethylcellulose, Alginsäure oder Carboxylmethylstärke, abgeleitet ist, und eine zweite Phase herzustellen, welche eines der Polymere aufweist.

13. Verfahren nach Anspruch 12, **dadurch gekennzeichnet, dass** es vorsieht, eines der Polymere in Form einer Polymerlösung herzustellen und zu reinigen, ein getrocknetes Pulver des vernetzten Hydrogels aus einem Teil der Polymerlösung herzustellen und das getrocknete Pulver des vernetzten Hydrogels mit einem getrockneten Pulver, welches aus einem anderen Teil der Polymerlösung gewonnen wurde, oder direkt mit dem anderen Teil der Polymerlösung in Verbindung zu bringen.

14. Verfahren nach Anspruch 13, **dadurch gekennzeichnet, dass** es vorsieht:
- die Polymerlösung durch teilweise Dehydration zu konzentrieren,
- die konzentrierte Polymerlösung durch eine teilweise Dehydration in zwei Fraktionen oder Teile zu teilen,
- das Lösungsmittel komplett von der ersten Fraktion oder dem ersten Teil zu entfernen,
- die erste Fraktion oder den ersten Teil, von welcher/m das Lösungsmittel entfernt wurde, einem Mahlen und Sieben zu unterziehen, um Partikel getrockneten Pulvers, welche eine homogene Verteilung des Nenndurchmessers haben, zu erhalten, um die erste Phase zu erhalten,
- eine zweite Fraktion oder einen zweiten Teil der konzentrierten Polymerlösung zu vernetzen, um das vernetzte Hydrogel, welches anschließend durch Waschen gereinigt wird, zu erhalten.
- das vernetzte Hydrogel zu vernetzen und die zum Reinigen des Hydrogels verwendete Waschflüssigkeit zu entfernen,
- das konzentrierte vernetzte Hydrogel, von welchem die Waschflüssigkeit entfernt wurde, einem Mahlen und Sieben zu unterziehen, um Partikel getrockneten Pulvers, welche eine homogene Verteilung des Nenndurchmessers haben, zu erhalten, um die zweite Phase zu erhalten,
- die erste Phase und die zweite Phase separat voneinander in einer Wasser- oder physiologischen Lösung zu dispergieren und anschließend die beiden die erste Phase und die zweite Phase enthaltenden Lösungen zusammenzusetzen.

15. Verfahren nach Anspruch 14, **dadurch gekennzeichnet, dass** es vorsieht, eine der Polymere in Form einer Polymerlösung herzustellen und zu reinigen, ein vernetztes Hydrogel in Lösung aus einem Teil der Polymerlösung herzustellen und das vernetzte Hydrogel in Lösung mit einem anderen Teil der Polymerlösung in Verbindung zu bringen.

16. Verfahren nach Anspruch 15, **dadurch gekennzeichnet, dass** es vorsieht:
- einen ersten Teil der Polymerlösung zu vernetzen, wobei das vernetzte Hydrogel erhalten wird, welches anschließend durch Waschen gereinigt wird,
- die Waschflüssigkeit, welche zur Reinigung des Hydrogels verwendet wurde, durch Trocknen zu entfernen,
- einen zweiten Teil der Polymerlösung mit dem getrocknetem Hydrogel in Kontakt zu bringen, wobei sichergestellt wird, dass das Polymer in die Netzstruktur des Hydrogels eingebracht ist,
- das Lösungsmittel zu entfernen,
- das erhaltene Produkt zu mahlen, um Pulver-Partikel mit einer gleichmäßigen Nominalverteilung zu erhalten.

17. Verfahren nach einem der Ansprüche 13 bis 16, **dadurch gekennzeichnet, dass** die Polymerlösung eines der hergestellten und verwendeten Polymere 1 Gew.-% bis 2 Gew.-%, bevorzugt 1,25 bis 1,75 Gew.-%, von einem ausgewählten der Polymere umfasst.

18. Verfahren zur Herstellung einer medizinischen Vorrichtung zur biomedizinischen Verwendung, welches vorsieht, eine gewünschte Menge des Verbundmaterials nach einem der Ansprüche 1 bis 9, oder erhältlich nach einem der Ansprüche 12 bis 17, in einer physiologischen Lösung oder in sterilem Wasser zu dispergieren.

## Revendications

1. Matériau composite convenant à un usage biomédical, **caractérisé en ce qu'**il comprend une première phase constituée d'un hydrogel réticulé dérivant d'un polymère choisi parmi les polymères qui sont des dérivés N-méthyl-amides de carboxy-méthyl-cellulose, l'acide alginique et l'amidon carboxyméthylé et une seconde phase qui comprend l'un desdits polymères.

2. Matériau selon la revendication 1, **caractérisé en ce qu'**il comprend de 2 à 5, de préférence de 2,5 à 4,5 et plus préférentiellement encore de 3 à 4 parties d'hydrogel réticulé et de 5 à 8, de préférence de 5,5 à 7,5, plus préférablement encore de 6 à 7 parties de polymère en tant que tel, à condition que la somme des parties des deux phases, sur une base pondérale, soit égale à 10.

3. Matériau selon la revendication 2, **caractérisé en ce que** le rapport entre la première phase et la seconde phase, sur une base pondérale, est choisi parmi 2:8, 2,5:7,5, 3:7, 3,5:6,5,4;6,4,5:5,5 et 5:5.

4. Matériau selon la revendication 1, 2 ou 3, **caractérisé en ce que** le degré d'occupation des fonctions carboxyle de l'hydrogel est supérieur à environ 70%.

5. Matériau selon l'une quelconque des revendications précédentes, **caractérisé en ce que** le polymère de la seconde phase est le même polymère réticulé que celui de la première phase.

6. Matériau selon l'une quelconque des revendications précédentes, **caractérisé en ce que** la solution de la seconde phase contient de 1% à 2% en poids, de préférence de 1,25% à 1,75% en poids, d'un polymère choisi parmi lesdits polymères.

7. Matériau selon l'une quelconque des revendications précédentes, **caractérisé en ce qu'**il contient en tout de 1,75% à 5% en poids, de préférence de 2% à 3,75% en poids, de l'un desdits polymères et dudit hydrogel réticulé dérivé de l'un desdits polymères.

8. Matériau selon l'une quelconque des revendications précédentes, **caractérisé en ce que** la première phase et la seconde phase sont formées de particules de poudre séchée avec une distribution homogène du diamètre nominal inférieur à ou égal à environ 2 mm.

9. Matériau selon l'une quelconque des revendications précédentes, **caractérisé en ce qu'**il comprend une solution aqueuse ou physiologique dans laquelle les première et seconde phases sont dispersées.

10. Produit comprenant une confection stérile contenant un matériau composite selon l'une quelconque des revendications précédentes.

11. Dispositif médical comprenant une dispersion d'une quantité désirée du matériau composite selon l'une quelconque des revendications précédentes dans une solution physiologique ou aqueuse.

12. Procédé d'obtention d'un matériau composite convenant à un usage biomédical, **caractérisé en ce qu'**il prévoit de préparer une première phase comprenant un hydrogel réticulé dérivant d'un polymère choisi parmi les polymères qui sont des dérivés N-méthyl-amides de carboxyméthylcellulose, l'acide alginique et l'amidon carboxyméthylé et une seconde phase qui comprend l'un desdits polymères.

13. Procédé selon la revendication 12, **caractérisé en ce qu'**il prévoit de préparer et purifier l'un desdits polymères sous la forme d'une solution de polymère, de préparer une poudre séchée d'hydrogel réticulé à partir d'une partie de ladite solution de polymère et d'associer ladite poudre séchée d'hydrogel réticulé avec une poudre séchée obtenue à partir d'une autre partie de ladite solution de polymère, ou directement avec ladite autre partie de solution de polymère.

14. Procédé selon la revendication 13, **caractérisé en ce qu'**il prévoit :
- de concentrer la solution de polymère par déshydratation partielle,
- de diviser la solution de polymère concentrée par déshydratation partielle en deux fractions ou parties,
- d'éliminer totalement le solvant d'une première fraction ou partie,
- de soumettre la première fraction ou partie, dont le solvant a été éliminé, à un broyage et à un tamisage de manière à obtenir des particules de poudre séchée qui ont une distribution homogène du diamètre nominal de façon à obtenir ladite première phase,
- de réticuler une seconde fraction ou partie de la solution de polymère concentrée, pour obtenir l'hydrogel réticulé qui est ensuite purifié par lavage,
- de concentrer l'hydrogel réticulé et d'éliminer le liquide de lavage utilisé pour purifier l'hydrogel,
- de soumettre l'hydrogel réticulé concentré dont le liquide de lavage a été éliminé à un broyage et à un tamisage de manière à obtenir des particules de poudre séchée qui ont une distribution homogène du diamètre nominal de façon à obtenir ladite seconde phase,
- de disperser dans une solution aqueuse ou physiologique, séparément l'une de l'autre, la première phase et la seconde phase et de mettre ensuite ensemble les deux solutions contenant la première phase et la seconde phase.

15. Procédé selon la revendication 14, **caractérisé en ce qu'**il prévoir de préparer et purifier l'un desdits polymères sous la forme d'une solution de polymère, de préparer un hydrogel réticulé en solution à partir d'une partie de ladite solution de polymère et d'associer ledit hydrogel réticulé en solution avec une autre partie de ladite solution de polymère.

16. Procédé selon la revendication 15, **caractérisé en ce qu'**il prévoit :
- de réticuler une première partie de la solution de polymère, pour obtenir l'hydrogel réticulé qui est ensuite purifié par lavage,
- d'éliminer le liquide de lavage utilisé pour purifier l'hydrogel, en séchant celui-ci,
- de mettre une seconde partie de la solution de polymère en contact avec l'hydrogel séché, en s'assurant que le polymère soit incorporé à l'intérieur de la structure réticulaire de l'hydrogel,
- d'éliminer le solvant,
- de broyer le produit obtenu afin d'obtenir des particules de poudre avec une distribution nominale homogène.

17. Procédé selon l'une quelconque des revendications 13 à 16, **caractérisé en ce que** la solution de polymère de l'un desdits polymères préparée et utilisée contient de 1% à 2% en poids, de préférence de 1,25 à 1,75% en poids, d'un polymère choisi parmi lesdits polymères.

18. Procédé d'obtention d'un dispositif médical pour un usage biomédical qui prévoit de disperser une quantité désirée de matériau composite selon l'une quelconque des revendications 1 à 9, ou que l'on peut obtenir selon l'une quelconque des revendications 12 à 17, dans une solution physiologique ou dans de l'eau stérile.
